# EUROPÄISCHE PATENTSCHRIFT

(11) **EP 3 705 041 B1**
(45) Veröffentlichungstag und Bekanntmachung des Hinweises auf die Patenterteilung: **25.06.2025**
(21) Anmeldenummer: 19160799.3
(22) Anmeldetag: 05.03.2019
(51) Int. Cl.: A61B 5/1455, A61B 5/00

(54) **SENSORANORDNUNG**
SENSOR ASSEMBLY
DISPOSITIF DE DÉTECTION

(43) Veröffentlichungstag der Anmeldung: 09.09.2020
(73) Patentinhaber: Luciole Medical AG, 8048 Zürich (CH)
(72) Erfinder: Fröhlich, Jürg Hans, 8006 Zürich (CH); Zahner, Marco, Zürich 8053 (CH)
(74) Vertreter: BOVARD AG

(56) Entgegenhaltungen:
- US-A- 6 014 576
- US-A1- 2009 182 209
- US-A1- 2014 051 956
- US-A1- 2016 029 890
- US-A1- 2017 319 132
- US-B1- 6 377 829

## Beschreibung

### Technisches Gebiet der Erfindung

Die vorliegende Erfindung bezieht sich auf eine Sensoranordnung, insbesondere eine Sensoranordnung zur nicht-invasiven Messung von Körperparametern, beispielsweise zerebralen Parametern wie den Sauerstoffgehalt des Gehirns.

### Hintergrund der Erfindung

Es ist bekannt, dass zur Messung von Körperparametern, insbesondere von zerebralen Parametern wie Konzentrationen von desoxygeniertem und oxygeniertem Hämoglobin des zerebralen Blutflusses oder des Gewebesauerstoffindexes, eine Messvorrichtung auf eine Körperoberfläche, z.B. eine Kopfoberfläche, angeordnet und eine Messung mittels Nahinfrarotspektroskopie (NIRS) durchgeführt wird.

Demnach kann durch Verwendung von Lichtquellen, welche Nahinfrarotlicht bei bestimmten unterschiedlichen Wellenlängen übertragen, und durch Messung von Änderungen der Extinktion detektierten oder reflektierten Lichts, Änderungen der Sauerstoffkonzentration von desoxygeniertem und oxygeniertem Hämoglobin überwacht werden. Mittels eines anderen spektrophotometrischen Verfahrens, der sogenannten Pulsoximetrie, ist durch Überwachung der pulsatilen optischen Extinktionsänderungen des detektierten Lichts, die arterielle Sauerstoffsättigung im peripheren Gewebe, beispielsweise Finger, Ohr, Nase, bestimmbar.

Es sind NIRS-Überwachungsanordnungen zur nicht-invasiven Überwachung, beispielsweise des Blutsauerstoffgehalts, bekannt, welche wiederverwertbare und zu entsorgende Komponenten umfassen. So ist zum Beispiel aus WO 94/27494 eine spektralphotometrische Sensormatte mit einer flexiblen Lage aus Schaummaterial zur Auflage auf einer Körperfläche bekannt, welche einen Trägerrahmen zur Aufnahme von Sensorelementen umfasst.

Aus EP 2 916 716 ist eine nicht-invasive Messvorrichtung zur Messung von Parametern eines Körpergewebes bekannt, welche eine Sensoreinheit und eine Sensormatte zum lösbaren Aufbringen auf einer Körperpartie bereitstellt. Die Sensoreinheit umfasst eine in einer Aufnahme aufgenommene Sensoranordnung mit einer an einer Unterseite in Richtung Körperoberfläche orientierten Sensorfläche, wobei die Sensoreinheit mittels eines Deckels oder einer Folie lichtdicht verschlossen wird. Die Aufnahme ist in einer an der biegsamen und/oder komprimierbaren Sensormatte ausgebildeten entsprechenden Aussparung aufgenommen, wobei die Sensormatte mit einer Auflagefläche auf der Körperoberfläche aufliegt und mittels einer Haftlage mit dieser verbindbar ist. Sensoreinheit, Sensormatte und Abdeckung sind lösbar miteinander verbunden, wobei Sensormatte und Abdeckung Einweg-Produkte sein können. Demnach umfasst die Sensoreinheit eine Sensoranordnung, aufgenommen in einer nur teilweise flexiblen Aufnahme, welche als eine Art flaches Gehäuse oder Schale ausgebildet ist, d.h. eine Grundfläche und eine Umfangswandung umfasst. Die Sensoranordnung weist eine daran angeordnete und fest mit dieser verbundenen Kontakteinheit für die Zu- und Ableitung elektrischer und optischer Signale, die Stromversorgung und die Verbindung mit einer externen Steuer- und Verarbeitungseinheit und/oder Kontrolleinheit auf.

Aus WO 2012/109661 ist eine NIRS-Sensoranordnung bekannt, welche einen Sensorteil mit einer Lichtquelle, beispielsweise eine Kombination aus faseroptischen Lichtleiter und Prisma und mindestens einem Lichtdetektor aufweist, ebenso wie eine flexible elektrische Schaltung und ein Verbinderelement. Die flexible elektrische Schaltung kann mehrschichtig sein und umfasst in einer Kommunikationsschicht mehrere Leiterbahnen und eine EMI-Abschirmung zur elektrischen Kontaktierung und Signalübertragung der Sensoranordnung mit dem Verbinderelement. Das Verbinderelement umfasst einen Faseroptik-Koppler als optische Schnittstelle und einen Leitungskoppler als elektrische Schnittstelle, wobei beide beispielsweise in einem Hybridverbinder zusammengefasst sein können. Eine lösbare Verbindung mittels Kontaktelementen eines derartigen Hybridverbinders an der Sensoranordnung wird nicht beschrieben.

Aus US 2009/182209 ist eine NIRS-Sensoranordnung bekannt, wobei von einer Lichtquelle mittels eines faseroptischen Lichtleiters und einem an der Sensoranordnung aufgenommenes, in einem Gehäuse angeordnetes Prisma Licht in eine Körpergewebe emittiert wird. Ferner erfolgt eine elektrische Kontaktierung der an einer flexiblen Schaltung angeordneten Lichtdetektoren der Sensoranordnung mittels eines abgeschirmten Kabels, welches auch unabhängig von der optischen Kontaktierung an der Sensoranordnung angeordnet sein kann. Die Kombination aus Lichtleiter und Prisma ist zwar drehbar an der Sensoranordnung angeordnet aber nicht mit dieser lösbar verbindbar.

Aus US 2017/319132 ist ein tragbares Kleidungsstück mit daran angeordneten Sensoren bekannt, welche eine Kommunikation mit einem tragbaren Telefon ermöglichen.

US 2016/029890 beschreibt ein System zur automatisierten Triage-Priorisierung, umfassend eine tragbare Einrichtung mit einer Vielzahl von Sensoren zur Erfassung von physiologischen, physikalischen und Umgebungsparametern. Demnach ist auch ein Pulsoximetriesensor vorgesehen mit Lichtemitter und Lichtdetektor, wobei die Einrichtung mittels vorgesehener Komponenten auf der Oberfläche in einer bestimmbaren Position angeordnet werden kann.

US 6,014,576 beschreibt eine zweiteilige Sonde mit einem universellen Sondenende und einem Verbindungskabelsegment, welches mit einer Vielzahl von verschiedenen photoplethysmographischen Geräten verwendet werden kann. Das Stecker-Ende des Verbindungskabelsegments ist jedoch starr, und kann nur in einer vorgegebenen Richtung in das Sondenende eingesteckt werden. Dadurch ist die Anbringung auf flache Körperoberflächen limitiert.

Aus dem Stand der Technik sind Messanordnungen bekannt, welche aufgrund einer mehr oder weniger starren Aufnahme für eine Sensoreinheit nicht über eine gewünschte Flexibilität verfügen, um auch an stark gekrümmten Körperflächen angeordnet werden zu können. Ferner ist auch die operative Kontaktierung der umfassten Sensoreinheit nicht unabhängig von der Messanordnung gelöst.

### Aufgabe der Erfindung

Es ist Aufgabe der vorliegenden Erfindung eine nicht-invasive Sensoranordnung zur Bestimmung von Körperparametern, bzw. Parametern des Körpergewebes, bereit zu stellen, bei welcher die Herstellungskosten gesenkt, die Flexibilität der Anordnung erhöht und die operative Kontaktierung unabhängig gestaltet ist. Da bei der Höhe der Herstellungskosten auch die Wiederverwertbarkeit zumindest einzelner Komponenten zu berücksichtigen ist, wird mit der Erfindung angestrebt, insbesondere teure Komponenten wiederverwertbar auszubilden. Insbesondere soll ein homogener Kontakt zwischen Sensoranordnung und Oberfläche des Körpergewebes sichergestellt und die Sensoranordnung zumindest teilweise wiederverwendbar sein.

Diese und weitere Aufgaben werden von einer nicht-invasiven Sensoranordnung gemäss dem unabhängigen Patentanspruch gelöst. Besondere Ausgestaltungen und/oder Varianten gehen aus den abhängigen Ansprüchen hervor.

Gemäss der Erfindung weist eine Sensoranordnung zur nicht-invasiven Messung von Parametern eine Sensoreinheit und eine Sensormatte zum lösbaren Aufbringen, bzw. zum Befestigen, der Sensoranordnung auf einer mehr oder weniger gekrümmten Körperoberfläche auf. Die Sensoranordnung umfasst einen Kontaktkopf, welcher mit der Sensoreinheit verbindbar ist, und welcher zum elektrischen Kontaktieren der Sensoreinheit und zum Zuführen von Licht von einer Lichtquelle zu der Sensoreinheit ausgebildet ist. Ferner ist die Sensoreinheit als eine flexible Leiterplatte ausgebildet mit optischen Komponenten, welche mindestens eine Lichtdetektoreinrichtung umfassen, um Licht zu detektieren, welches von dem an der Sensoreinheit aufgenommenen und verbundenen Kontaktkopf in das Körpergewebe emittiert und dieses passiert hat, sowie einem ersten Kontaktmittel und Leiterbahnen zum operativen Verbinden der Sensoreinheit mit dem Kontaktkopf. Die Sensoreinheit ist zudem als entlang einer Längsachse gestreckter Körper mit Laschen ausgebildet, welche im Wesentlichen senkrecht zur und/oder entlang der Längsachse ausgebildet sind. Hierbei sind an dem langgestreckten Körper der Sensoreinheit im Bereich der optischen Komponenten, bzw. der Lichtdetektoreinrichtung, Laschen ausgebildet, welche sich senkrecht und/oder entlang der Längsachse erstrecken. Die Sensormatte der erfindungsgemässen Sensoranordnung hat einen mehrschichtigen Aufbau, mit einer unteren Schicht, welche eine untere Auflagefläche zur Auflage auf der Körperoberfläche und eine obere Auflagefläche zur Auflage der Sensoreinheit aufweist, wobei Aussparungen vorgesehen sind, welche auf die optischen Komponenten der Sensoreinheit abgestimmt sind, und einen optischen Durchgang bilden. An einer Unterseite der unteren Schicht und damit der Körperoberfläche zugewandt kann ferner zumindest eine weitere Schicht angeordnet sein, welche in den Bereichen der Aussparungen optisch transparente Abschnitte aufweist und demnach eine Art Folienfenster in Bezug zu den Lichteinkopplungs- bzw. Lichtauskopplungsabschnitten bildet. Optisch transparent bedeutet, dass durch eine derartige Schicht eine Lichtmenge treten kann, welche beispielsweise für eine NIRS-Analyse ausreichend ist. Andere Bereiche der Schicht können dagegen optisch nicht transparent sein. Alternativ können eine erste optisch transparente Schicht und eine weitere optisch nicht transparente Schicht mit entsprechenden optischen Durchgängen an der Unterseite der unteren Schicht der Sensormatte angeordnet sein. Die optisch nicht transparenten Abschnitte, bzw. die optisch nicht transparente weitere Schicht, stellen eine Art Isolierschicht bereit, welche zwischen den optisch aktiven Bereichen der Sensoranordnung angeordnet sind und demnach eine Beeinflussung der Lichtdetektion durch beispielsweise seitlichen Lichteinfall und/oder Hintergrundlicht verhindern. Eine derartige Abdeckung der optisch aktiven Komponenten wirkt sich vorteilhaft im Hinblick auf eine sterile Sensoranordnung aus. Ferner umfasst die Sensormatte eine obere Schicht, welche mit der oberen Auflagefläche der unteren Schicht voneinander lösbar verbunden ist. Eine Aussenform der Sensoranordnung ist mit Einkerbungen bzw. Einschnitten gestaltet, so dass diese an unterschiedlichsten Körperoberflächen anordenbar ist und insbesondere einen guten Tragekomfort bietet.

Die Sensoranordnung besteht aus voneinander trennbaren Komponenten, wobei der Kontaktkopf und die Sensoreinheit eine operative Verbindung eingehen und die Sensormatte ausgebildet ist, um die damit verbundene Sensoreinheit leicht und sicher an der Haut einer zu untersuchenden Person zu befestigen. Es ist wirtschaftlicher, die Sensoreinheit und/oder den Kontaktkopf als wiederverwendbare Elemente zu gestalten, während gleichzeitig die hohen Anforderungen an Hygiene und Sterilisation bei der Anwendung im Gesundheitswesen erfüllt werden. Die Sensoreinheit ist demnach in der mehrschichtigen Sensormatte aufnehmbar, deren einzelne Schichten voneinander trennbar sind, so dass die dazwischen aufgenommene Sensoreinheit entnommen und nach vorbereitenden Schritten wieder verwendet werden kann.

Die Sensoreinheit einer derartigen optoelektronischen Sensoranordnung ist gemäss der Erfindung als flexible oder starr-flexible Leiterplatte ausgebildet, auch bezeichnet als Flexprint. An der Sensoreinheit können Ausgänge einer oder mehrerer Lichtquellen, wie etwa Leuchtdioden, Laserdioden oder eine oder mehrere Lichtquellen selbst angeordnet sein und davon beabstandet eine oder mehrere Sensorflächen, z.B. als Fotodioden ausgebildete Lichtdetektoren, ebenso wie elektrische Leitungen, die z.B. Licht der Lichtquelle oder der Lichtquellen oder elektrische Signale von und zu den Elementen der Sensoreinheit leiten. Ebenfalls können weitere Elemente vorgesehen sein, beispielsweise eine Kontrolleinheit, welche über entsprechend vorgesehenen Sensorflächen eine Hintergrundbeleuchtung jeweils zwischen einzelnen Lichtpulsen der Lichtquelle misst und/oder den elektrischen Strom. Demnach kann die Kontrolleinheit über die Sensorflächen automatisch eine Hintergrundbeleuchtung jeweils zwischen Laserlichtpulsen messen, ausgehend von der Lichtquelle, welches als Korrektur des gemessenen Lichts während der Laserlichtimpulse genutzt wird. Bei einer teilweise oder vollständig abgelösten Sensoranordnung verändert sich die Hintergrundbeleuchtung, so dass entweder ein Störsignal oder eine Störautomatik erzeugt werden kann, welche zur automatischen Abschaltung der Leucht- bzw. Laserdioden führen kann. Ferner kann die Kontrolleinheit für weitere Kalibrierungs- und Messaufgaben verwendet werden.

Als Ergänzung kann ferner ein Mittel zur Erfassung von Temperatur und/oder Wärmefluss vorgesehen sein. Beispielsweise kann vorgesehen sein, die Temperatur des zu untersuchenden Gewebes mittels NIRS zu ermitteln, welches auf einer Temperaturabhängigkeit des Wasserabsorptionsspektrums basiert. Ferner kann aber auch eine der umfassten Sensorflächen verwendet werden, um die Temperatur einer in der Sensoranordnung integrierten Lichtquelle zu erfassen zur Verifizierung des Temperatureinflusses auf die Emissionscharakteristiken der Lichtquelle, z.B. der LED's, und der ermittelten Messergebnisse. Es ist bekannt, dass Wärme von einer nahe an der Körperoberfläche positionierten Lichtquelle von einem Patienten als unangenehm empfunden wird, bzw. bei zu hoher Temperatur es sogar zu einem Ausfall einzelner Komponenten der Sensoranordnung kommen kann.

Vorzugsweise werden als Lichtquelle wenigstens vier verschiedene Leucht- bzw. Laserdioden unterschiedlicher Wellenlänge vorgesehen, die zeitlich gestaffelt ein- und ausgeschaltet werden können. Die Lichtquelle ist selektiv betreibbar, um insbesondere infrarotes Licht zu leiten oder zu emittieren, wobei die Lichtquelle entweder als eine in der Sensoranordnung integrierte Lichtquelle selbst Lichtsignale erzeugt oder Lichtsignale an einer Position ausserhalb der Sensoranordnung erzeugt und mittels einer Faseroptik der Sensoranordnung zugeführt werden. So können eine oder mehrere Lichtquellen, ausgebildet als Leuchtdioden oder Laserdioden, in dem Kontaktkopf direkt integriert sein, so dass Verluste der Leuchtdichte an optischen Kopplungsstellen vermieden werden. Diese integrierten Lichtquellen werden als interne Lichtquellen bezeichnet.

Übertragungsverluste werden weitgehend vermieden, wenn von einer entfernten Lichtquelle das Licht mittels Faseroptik via dem Kontaktkopf in das zu untersuchende Körpergewebe emittiert wird. Insbesondere bei einer ausserhalb der Sensoranordnung positionierten Lichtquelle, d.h. einer externen Lichtquelle, wird vorteilhaft vermieden, dass es zu einer Beeinflussung des Untersuchungsraums bzw. des Blutflusses durch Wärmeentwicklung der Lichtquelle kommen kann.

Die Lichtdetektoren, insbesondere die eine oder mehrere Fotodioden, sind an der Sensoreinheit angeordnet, so dass dieser von der Lichteinkopplungsstelle, um einige mm bis mehr als 20 mm getrennt ist. Vorzugsweise beträgt ein Abstand von der Lichteinkopplungsstelle zu mindestens einer ersten Fotodiode, bezeichnet als Nahdetektoren, etwa 20 mm und zu einer oder mehreren weiteren zweiten Fotodioden etwa 40 mm, welche als Ferndetektoren bezeichnet sein können. Dabei können sich die relative Position und der Abstand der Lichtdetektoren von der oder den Lichteinkopplungsstellen nach der Grösse des zu untersuchenden Objekts richten. Die Anzahl der Fotodioden können in den Lichtdetektoren je nach Anwendung variieren. Mehrere Fotodioden können verwendet werden, um verschiedene Tiefen der Blutoxygenierung in dem Subjekt zu überwachen, oder können als Referenzdetektoren verwendet werden, beispielsweise um in einem Algorithmus Störeffekte der detektierten Signale zu kompensieren.

In Bezug auf die Sensoreinheit wird als Flexprint allgemein eine flexible oder starr flexible Leiterplatte mit gedruckten Leiterbahnen mit einem Substrat aus einem isolierenden Polymermaterial mit daran angeordneten bzw. eingebetteten Fasern aus einem elektrisch leitenden Material zur Ausbildung einer flexiblen Schaltung bezeichnet. Eine flexible Schaltung in Zusammenhang mit einem flexiblen Substrat verbessert die Flexibilität einer derartigen Sensoreinheit. Die als flexible Leiterplatte ausgebildete Sensoreinheit kann mehrschichtig aufgebaut sein, insbesondere mit einer Kommunikationsschicht, beispielsweise umfassend ein flexibles kupferkaschiertes Laminat, sowie mit Deckschichten bzw. Isolierschichten und entsprechenden Klebeschichten ausgebildet sein. Die mehreren Schichten der Sensoreinheit können laminiert, zusammengefügt oder auf andere Weise miteinander verbunden sein, um eine einzige Struktur zu bilden. Die Anzahl der Schichten kann ebenfalls variieren. Beispielsweise können eine oder mehrere Schichten optisch transparente Abschnitte zumindest in Ausrichtung zu den Bereichen der Lichteinkopplungsstelle und der Lichtdetektorflächen aufweisen, durch welche eine Lichtmenge hindurchtreten können. Zum Beispiel kann der optisch transparente Abschnitt ein elektrisch leitendes Drahtgeflecht, z.B. ein Kupferdrahtgeflecht, enthalten. Andere Abschnitte der Sensoreinheit sind optisch nicht transparent und können beispielswiese aus einer Kupfermetallfolie gebildet sein. Eine derartig ausgebildete Sensoreinheit zeichnet sich durch ein geringes Gewicht und Volumen aus, bietet eine hohe Designfreiheit und dynamische und mechanische Belastbarkeit.

In einer Ausführungsform sind zumindest abschnittsweise an der Sensoreinheit Verstärkungselemente vorgesehen. Diese auch als Stiffener bezeichnete Verstärkungselemente können als Formstücke aus einen starren Material ausgebildet sein, insbesondere in Form von Polymerverdickungen, die in gewünschten Bereichen, beispielsweise im Bereich der Fotodioden, die flexible Leiterplatte versteifen. Zur weiteren Stabilisierung der Form und der Planheit der Sensoreinheit kann ein Kupfernetz als Flechtschicht vorgesehen sein. Das vorgesehene Kupfernetz kann auch eine Art Abschirmung gegen elektromagnetische Interferenzen bereitstellen.

Die Sensoreinheit ist als ein sich entlang einer Längsachse erstreckender Körper ausgebildet. Die Formgebung der Sensoreinheit berücksichtigt abgerundete Übergänge und entsprechend geformte Abschnitte, so dass Bruchstellen vermieden werden, wenn die Sensoreinheit in einer starken Krümmung oder stark gebogen angeordnet wird, beispielsweise um einen Finger gelegt ist.

Die als langgestreckter Körper ausgebildete Sensoreinheit weist Laschen auf, welche sich im Wesentlichen senkrecht zu beiden Seiten und/oder entlang der Längsachse des Körpers erstrecken. Die Laschen, auch als Nasen bezeichnet, sowie die grundsätzliche Formgebung des Körpers der Sensoreinheit erlauben seine plane Auflagefläche auf der Sensormatte. Hierbei liegen die optischen Komponenten, d.h. Lichtdetektoreinrichtungen und/oder Emitter, beispielsweise auf optisch transparenten Flächen bzw. Fenstern der Sensormatte oder direkt an der Körperoberfläche für eine effiziente Messung auf. Mit der erfindungsgemässen Ausgestaltung der Sensoreinheit ist es nicht erforderlich, dass die Sensoreinheit in einer mehr oder weniger starren Aufnahme aufgenommen und darin mittels Silikonguss fixiert wird, um dann an oder in einer Sensormatte aufgenommen zu werden. Die Flexibiltät erhöht sich dementsprechend.

Gemäss einer Ausführungsform umfasst die Sensoreinheit die ersten Kontaktmittel mit einer Kontaktgeometrie, welche zum elektrischen Kontaktieren und zum Verbinden mit dem Kontaktkopf ausgebildet ist. Demnach stellt das erste Kontaktmittel eine operative Verbindung zur elektrischen Versorgung der Sensoreinheit und zur Verbindung mit einer Lichtquelle bereit, ebenso wie zur Übermittlung von detektierten Signalen. Mit anderen Worten ist das erste Kontaktmittel ein Kopplungselement bzw. ein Steckerelement, welches mit einem entsprechend ausgebildeten Gegenstück lösbar verbunden werden kann. Die Verbindung kann unteranderem als Steckkontakt oder Rastung ausgebildet sein. In einer bevorzugten Ausführungsform umfasst die Kontaktgeometrie hierfür magnetische und elektrische Kontaktelemente. So kann das erste Kontaktmittel als ein Kontaktring ausgebildet sein, welcher an der als flexible oder starr-flexible Leiterplatte ausgebildete Sensoreinheit in einer Position ausgebildet ist, welche geeignet ist, um mit dem noch näher zu erläuternden Kontaktkopf in Kontakt gebracht zu werden. Insbesondere kann der Kontaktring die Form eines PCB-Rings haben, umfassend eine Vielzahl von Elementen zur elektrischen und mechanischen bzw. magnetischen Kontaktierung mit entsprechenden an dem Kontaktkopf vorgesehenen Gegenstücken. Dabei ist vorgesehen, dass der verbindbare Kontaktkopf in einer vorbestimmbaren Orientierung relativ zur Sensoreinheit positionierbar ist. Insbesondere kann eine Ausrichtungshilfe, beispielsweise in Form eines Positionierrings, an dem Kontaktring angeordnet sein, welcher durch eine asymmetrische Formgebung bzw. Formabschnitten eine vorbestimmte Ausrichtung des verbindbaren Kontaktkopfes vorgibt. So können Kabel, welche zur elektrischen und/oder optischen Verbindung an dem Kontaktkopf verbunden sind, in einer Orientierung ausgerichtet werden, dass eine Person durch die befestigte Sensoranordnung nicht gestört wird. Insbesondere kann der Positionierring mit der Sensoreinheit verklebt sein.

Das erste Kontaktmittel weist die Kontaktgeometrie auf, umfassend elektrische und magnetische Kontaktelemente. Insbesondere können die Kontaktelemente auf einem Kontaktring zusammengefasst sein, wobei die Kontaktgeometrie auf einer Ringfläche in einer Anordnung vorgesehen ist, so dass beispielsweise mehrere elektrische Kontaktelemente, insbesondere Kontaktpunkte, zur elektrischen Kontaktierung der Sensorflächen, d.h. der Fotodioden der Nah- und/oder Ferndetektoren und ein oder mehrere magnetisch wirksame Kontaktelemente vorhanden sind. Die magnetisch wirksamen Kontaktelemente können als Weissblechpunkte ausgebildet sein. Vorzugsweise sind elektrische und magnetische Kontaktelementen in einer sich gegenseitig abwechselnden Reihenfolge positioniert, d.h. dass ein elektrisches Kontaktelement zwischen benachbarten magnetischen Kontaktelementen liegt. Hiermit wird eine gegenseitige Beeinflussung elektrischer Kontaktelemente vermieden. Die magnetischen Kontaktelemente sind so ausgewählt, dass eine sichere Kontaktierung zwischen Sensoreinheit und Kontaktkopf vorliegt. Ferner kann vorgesehen sein, dass die sichere Kontaktierung von Sensoreinheit und Kontaktkopf mittels entsprechender Erfassungsmittel bzw. Kontrollmittel detektiert und angezeigt wird.

Die Sensoreinheit der erfindungsgemässen Sensoranordnung kann in einfacher Weise hergestellt werden, in dem vorgefertigte Bauteile lediglich zueinander in Verbindung gebracht werden müssen, vorzugsweise durch "pick und place"-Schritte.

Die Sensoreinheit und der damit operativ verbindbare Kontaktkopf können mit der Sensormatte lösbar verbunden werden. Die Sensormatte weist Ausnehmungen, bzw. Öffnungen, oder transparente Fenster auf, die entsprechend der optischen Komponenten der Sensoreinheit, d.h. Lichtdetektoreinrichtung und/oder Emitter, angeordnet und ausgebildet sind. Grundsätzlich ist die Sensormatte als eine längliche flache Matte ausgebildet, mit einem mehrschichtigen Aufbau, wobei beispielsweise mindestens eine Schaumschicht aus einem dünnen, biokompatiblen Schaummaterial mit einer bzw. beidseitig angeordneter Klebeschicht umfasst ist.

Hierbei ist eine untere Schicht umfasst, welche Öffnungen bzw. Durchgänge oder auch transparente Fenster im Bereich der Sensorflächen, d.h. der Fotodioden, und dem Einkopplungsbereich, bzw. der Lichtaustrittsfläche des Lichts, d.h. dem Emitter, aufweist und welche mit einer Unterseite mit einer Körperoberfläche in Kontakt steht und an dieser mittels eine Klebeschicht aufbringbar ist. Die entsprechende Oberseite ist ebenfalls klebend, so dass die darauf aufliegende Sensoreinheit insbesondere mittels der daran ausgebildeten Laschen bzw. Nasen gehalten ist. Desweiteren ist mindestens eine weitere Schicht, d.h. eine obere Schicht, umfasst, welche nur eine Aussparung im Bereich der Lichteinkopplung, d.h. im Bereich des Kontaktkopfes, aufweist. Die obere Schicht umfasst eine untere Klebschicht, welche mit der Oberseite der unteren Schicht in einer haftenden Verbindung steht, so dass dazwischen die Sensoreinheit positionssicher aufgenommen ist. Die mehrschichtige Sensormatte kann als Einweg-Einheit ausgebildet sein, d.h. nach einer Messung kann die Sensoranordnung von der Körperoberfläche entfernt und die einzelnen Komponenten, d.h. Sensoreinheit, Sensormatte und Kontaktkopf, voneinander getrennt und die Sensormatte entsorgt bzw. wieder aufbereitet werden.

Vorzugsweise ist die Sensormatte flexibel, bzw. biegsam ausgebildet, so dass die Sensormatte an die Körperoberfläche anpassbar ist und sich an Krümmungen, Erhebungen oder Vertiefungen derselben anpasst. Die Gestaltung der Sensormatte sieht einen Aussenumfang vor, welcher beispielsweise Einkerbungen und/oder Dünnstellen aufweist, welche entlang des gesamten Aussenumfangs vorgesehen sind. Insbesondere kann der Aussenumfang an den Bereichen, an denen die Laschen bzw. Nasen der Sensoreinheit an der Sensormatte zu liegen kommen, in Form von Flügeln ausgebildet sein, so dass eine leichte Anpassung der Sensormatte an die Kontur der Körperfläche ermöglicht wird.

In einer alternativen Ausführungsform der Sensormatte kann zwischen der unteren Schicht und der oberen Schicht eine mittlere Schicht vorgesehen sein.

Zur Messung von Körperparametern wird in einer Ausführungsform die Sensoranordnung dadurch aktiviert, dass mittels des mit der Sensoreinheit verbundenen Kontaktkopfes eine Einkopplung von Lichtwellen und eine elektrische Kontaktierung erfolgen. Der mit der Sensoreinheit verbindbare Kontaktkopf umfasst ein Gehäuse, mindestens zweite Kontaktmittel, welche eine Kontaktgeometrie entsprechend dem ersten Kontaktmittel der Sensoreinheit aufweisen, elektrische Leitungen, welche in dem Gehäuse fixierbar und welche mit elektrischen Kontaktelementen der zweiten Kontaktmittel leitend verbindbar sind. Der Kontaktkopf umfasst Mittel zum Bereitstellen und Führen von Licht in einer Richtung, welche senkrecht zur Körperoberfläche gerichtet ist und eine Lichtaustrittsfläche, über die Licht in das Körpergewebe emittiert. In einer Ausführungsform umfassen die Mittel eine Faseroptik, um Licht von einer externen Lichtquelle zuführen und ein Umlenkelement, um das in einer Richtung zugeführte Licht in die Richtung senkrecht zur Körperoberfläche umzulenken. Das Umlenkelement kann eine Reflexionsfläche bereitstellen, um das zugeführte Licht aus einer ersten Richtung in eine zweite Richtung umzulenken, welche im Wesentlichen senkrecht zu der Körperoberfläche gerichtet ist, und via einer Lichtaustrittsfläche in das Körpergewebe emittiert.

In einer alternativen Ausführungsform ist vorgesehen, dass die Mittel zum Bereitstellen und Führen von Licht eine interne Lichtquelle umfassen, beispielsweise in Form von einer oder mehrerer Leucht- bzw. Laserdioden, welche in dem Kontaktkopf selbst als interne Lichtquelle integriert ist, und ein Lichtleitelement, so dass davon ausgehendes Licht in Richtung senkrecht zur Körperoberfläche gelenkt wird und über eine Lichtaustrittsfläche emittiert. Demnach ist in einer derartigen Ausführungsform keine Faseroptik erforderlich, sondern die von der internen Lichtquelle ausgehenden Lichtwellen werden mittels des Lichtleitelements via der Lichtaustrittsfläche in das damit in Kontakt stehende Körpergewebe emittiert.

Ferner kann vorgesehen sein, dass zur elektrischen Kontaktierung der in dem Kontaktkopf integrierten internen Lichtquelle und/oder der mit dem Kontaktkopf verbindbaren Sensoreinheit eine in dem Kontaktkopf aufgenommene Energiequelle vorgesehen ist, so dass die elektrischen Leitungen innerhalb des Kontaktkopfes verlaufen zur elektrischen Kontaktierung der mindestens einen Lichtdetektoreinrichtung der Sensoranordnung, während keine externen Verbindungen erforderlich sind. Eine Übertragung der Signale, d.h. eine Datenübertragung, von der mindestens einen Lichtdetektoreinrichtungen und/oder Kontrolleinheit zu einer externen Steuer- und/oder Verarbeitungseinheit kann mittels drahtloser Kommunikation erfolgen.

Vorgesehene Kontrollmittel stellen sicher, dass kein Licht emittiert wird, wenn kein Kontakt zu einer Körperoberfläche besteht, bzw. wenn der Kontaktkopf nicht sicher mit der Sensoreinheit verbunden ist. Hierfür sind Kontrollmittel vorgesehen, um eine Hintergrundbeleuchtung zu messen, wobei die Messung der Hintergrundbeleuchtung genutzt wird, um eine Not-Ausschaltung der Sensoranordnung zu realisieren. Sobald der Wert bzw. die Intensität der Hintergrundbeleuchtung einen vorbestimmten Maximalwert überschreitet, werden die eine oder mehreren Lichtquellen ausgeschaltet, um sicherzugehen, das keine Gefahr besteht, falls die Sensoranordnung sich teilweise oder vollständig von der Körperoberfläche absichtlich oder unabsichtlich gelöst hat.

Die elektrischen Leitungen und die Zuführungen von Licht, z.B. als Faseroptik mit mindestens einer optischen Faser ausgebildet, können in einem Hybridkabel zusammengefasst sein, welches an dem Kontaktkopf fixierbar ist.

Zur elektrischen Kontaktierung der Sensoreinheit der erfindungsgemässen Sensoranordnung sind die zweiten Kontaktmittel vorgesehen, welche in dem Gehäuse des Kontaktkopfes als separate ringförmige Komponenten aufnehmbar sind. Die zweiten Kontaktmittel umfassen in einer Ausführungsform einen ersten Kontaktring, an welchem auf einer ersten Seite Federkontaktstifte in einer vorgegebenen Anordnung und auf einer zweiten Seite Steckerelemente angeordnet sind, welche mit einem an den elektrischen Leitungen vorgesehenen Gegenstück einen Steckerkontakt bilden, und einen zweiten Kontaktring, welcher Durchgangsbohrungen aufweist, durch welche sich die Federkontaktstifte erstrecken und an welchem auf einer ersten Seite magnetische Kontaktelemente angeordnet sind, welche mit denjenigen des ersten Kontaktmittels in Kontakt bringbar sind.

Demnach sind die zweiten Kontaktmittel mit einer zu dem ersten Kontaktmittel komplementären Kontaktgeometrie ausgebildet. Die zweiten Kontaktmittel können ringförmig gestaltet sein, insbesondere als im Wesentlichen ringförmige Leiterplatine, d.h. als PCB, wobei zur elektrischen Kontaktierung an einer ersten Seite Verbindungselemente, vorzugsweise Federkontaktstifte vorgesehen sind. Die Verbindungselemente sind derart an den zweiten Kontaktmitteln auf einer Seite in einer Anordnung positioniert, dass sie mit einer vorgegeben Orientierung in Kontakt mit den elektrischen Kontaktelementen des ersten Kontaktmittels gebracht werden können, wobei die exakte Orientierung durch eine Formgebung der zweiten Kontaktmittel bereitgestellt werden kann. Die zweiten Kontaktmittel können an einer Unterseite des Kontaktkopfes, bzw. des Gehäuses, platziert sein, d.h. in etwa parallel zur Körperoberfläche. Die an der zweiten Seite und damit gegenüberliegend der mit den Federkontaktstiften aufweisenden ersten Seite sind an den zweiten Kontaktmitteln Steckerelemente angeordnet, insbesondere ein Mikrostecker. Demnach können die elektrischen Leitungen via einer Steckerverbindung mit den Federkontaktstiften in leitenden Kontakt gebracht werden.

Ferner umfassen die zweiten Kontaktmittel den zweiten Kontaktring, welcher auf einer ringförmigen Fläche eine entsprechende Anordnung von Magnetelementen und Durchgangsbohrungen aufweist, welche so ausgebildet sind, dass durch diese die Verbindungselemente, d.h. die Federkontaktstifte, aufgesteckt werden können, so dass sie über die Ringfläche vorstehen. Insbesondere wechseln sich auf der Ringfläche des zweiten Rings Durchgangslöcher und Magnetelemente ab, so dass eine sichere elektrische und magnetische Kontaktierung des Kontaktkopfes mit den Gegenstücken am ersten Kontaktmittel der Sensoreinheit bereit gestellt ist.

In einer Ausführungsform mit einer externen Lichtquelle ist an dem Kontaktkopf eine Faseroptik fixierbar, welche mindestens eine optische Faser umfasst, die in dem Gehäuse oder einem in dem Gehäuse aufnehmbaren Element fixiert ist. Demnach stellt der Kontaktkopf neben der elektrischen Kontaktierung auch eine Führung von Licht beispielsweise von einer entfernten Lichtquelle und ein Einkoppeln der Lichtwellen in das zu untersuchende Gewebe bereit. Entlang der Faseroptik wird Licht, z.B. Nahinfrarotlicht, entlang einer ersten Richtung gerichtet und an einer Reflexionsfläche umgelenkt, so dass das Licht entlang einer zweiten Richtung, im Allgemeinen senkrecht zur ersten Richtung und insbesondere senkrecht zur Körperoberfläche, aus dem Kontaktkopf an der Lichtaustrittsfläche austritt. Demnach wird Licht aus dem Kontaktkopf heraus auf ein Objekt projiziert, beispielsweise über ein entsprechend ausgebildetes Einkoppelelement, welches in unmittelbaren Kontakt mit der Körperoberfläche gebracht werden kann. Die Faseroptik umfasst ein oder mehrere optische Fasern, welche in einer Bohrung in dem Gehäuse des Kontaktkopfes oder einem darin aufnehmbaren Halteelement aufgenommen und darin gehalten ist/sind. Das Haltelement kann in direktem Kontakt mit einem Umlenkelement stehen, das die Reflexionsfläche bereitstellt, so dass Lichtstrahlen entsprechend umgelenkt werden und in das zu untersuchende Gewebe weitgehend senkrecht über einen flächigen Kontakt emittieren. Beispielsweise kann das Umlenkelement als Prisma ausgebildet sein.

Das Umlenkelement kann als Zylinder mit einer geschlossenen abgeschrägten Endfläche ausgebildet ist. Hierfür ist das Umlenkelement als galvanisiertes Kunststoffteil ausgebildet, wobei die aus der Faseroptik austretenden Lichtwellen an der bereitgestellten Reflexionsfläche in Richtung Lichtaustrittsfläche umgelenkt und/oder reflektiert werden.

Alternativ ist das Umlenkelement als Hohlzylinder mit einer geschlossenen halbkugelförmigen Endfläche ausgebildet. Das Umlenkelement ist insbesondere aus einem reflektierenden Material gefertigt. Hierbei mündet die Bohrung zur Aufnahme der Faseroptik an der halbkugelförmigen Endfläche, wobei austretendes Licht in Richtung der zweiten Richtung und damit in Richtung Lichtaustrittsfläche an der halbkugelförmigen Reflexionsfläche umgelenkt wird.

Gemäss der Erfindung ist der zur Sensoreinheit komplementäre Kontaktkopf als eine separate Einheit gefertigt, so dass eine optimierte Herstellung unabhängig von den weiteren Komponenten der Sensoranordnung möglich ist. Lediglich die Kontaktmittel von Kontaktkopf und Sensoreinheit werden aufeinander abgestimmt. Demnach ist auch eine Kontaktierung der Sensoreinheit der erfindungsgemässen Sensoranordnung in einfacher Weise möglich, ebenfalls unter Berücksichtigung örtlicher Gegebenheiten, um die Orientierung der Zu- und Ableitungen für einen Patienten so angenehm wie möglich zu gestalten.

### Kurze Beschreibung der Zeichnungen

Bevorzugte Ausführungsformen der Erfindung werden im Folgenden anhand der Zeichnungen dargestellt, die lediglich der Erläuterung dienen und nicht einschränkend auszulegen sind. Der Schutzumfang der vorliegenden Erfindung wird durch die Ansprüche definiert.

In den Zeichnungen zeigen:
Fig.1 eine schematische Darstellung als Schnittansicht durch eine Sensoranordnung nach der vorliegenden Erfindung,
Fig. 2a eine Sensoreinheit einer erfindungsgemässen Sensoranordnung, ausgebildet als flexible Leiterplatte mit dem ersten Kontaktmittel zur elektrischen und optischen Kontaktierung,
Fig. 2b einen Positionierring, anordenbar an der Sensoreinheit gemäss Fig. 2a,
Fig. 3 eine untere Ansicht einer Sensormatte einer erfindungsgemässen Sensoranordnung,
Fig. 4 eine obere Ansicht einer Sensormatte einer erfindungsgemässen Sensoranordnung,
Fig. 5 eine Detailansicht als Schnittansicht eines Kontaktkopfs gemäss einer Ausführungsform,
Fig. 6 eine Detailansicht als Schnittansicht eines Kontaktkopfes in einer zweiten Ausführungsform.
Fig. 7 eine Detailansicht eines Umlenkelements des Kontaktkopfs gemäss einer Ausführungsform.

### Detaillierte Beschreibung der Ausführungsformen der Erfindung

In der folgenden Beschreibung einer Sensoranordnung nach der vorliegenden Erfindung soll eine Unterseite als eine einer Körperoberfläche zugewandte Seite und eine Oberseite als eine der Unterseite gegenüberliegende Seite verstanden werden. Dabei sind Ober- und Unterseiten im Wesentlichen zumindest annähernd parallel zu einer Körperoberfläche bzw. zumindest abschnittsweise parallel.

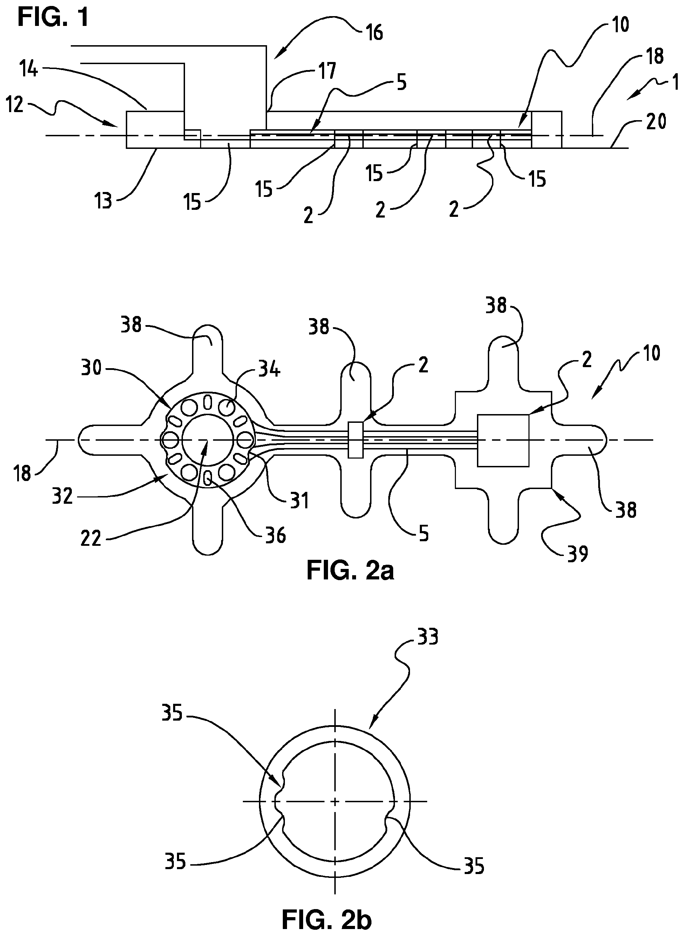

In Figur 1 ist in schematischer Weise der Aufbau einer Sensoranordnung 1 nach der Erfindung zur nicht-invasiven Messung von Parametern eines Körpergewebes mit einer Sensoreinheit 10 und einer Sensormatte 12 zum lösbaren Aufbringen der Sensoranordnung 1 auf einer Körperoberfläche 20 gezeigt.

In der dargestellten Ausführungsform umfasst die Sensoranordnung 1 im Wesentlichen die wiederverwendbare Sensoreinheit 10, die Sensormatte 12 und einen Kontaktkopf 16 zur elektrischen und optischen Kontaktierung der Sensoranordnung 1. Die Sensoreinheit 10 weist von der Position des Kontaktkopfs 16 beabstandete Lichtdetektoreinrichtungen 2, auch als Messflächen 2 bezeichnet und Leiterbahnen 5 auf. Weitere Elemente, beispielsweise Kontrolleinheiten, können in der Sensoranordnung 1 vorgesehen werden. Von einer Lichtquelle kann über den Kontaktkopf 16 oder über eine direkt an der Sensoreinheit 10 bzw. in dem Kontaktkopf 16 angeordnete Lichtquelle mit mehreren Leucht- bzw. Laserdioden, vorzugsweise vier Laserdioden, Licht mit jeweils unterschiedlichen Wellenlängen im nahinfraroten Bereich (NIRS) zur Messung von Parametern emittiert werden, vorzugsweise zeitlich gestaffelt. Als Sensorflächen 2 werden z.B. Fotodioden verwendet. Die Sensoreinheit 10 ist entlang einer Längsachse 18 in einer länglichen, flachen Körperform gestaltet, mit einer definierten Aussenkontur, welche auf die Aussenkontur der umfassten Elemente und auf die aufzubringende Körperoberfläche abgestimmt ist. Die Sensoreinheit 10 ist als flexible Leiterplatte ausgebildet und demnach bis zu einem gewissen Grad biegbar. Die Sensoreinheit 10 ist an der Sensormatte 12 aufgenommen, welche eine unter Schicht 13 und eine obere Schicht 14 umfasst.

Die untere Schicht 13 weist auf einer Unterseite eine Grundfläche mit mehreren Aussparungen 15 auf, welche einen Durchgang für Licht bereitstellen. Die Grundfläche ist der Körperoberfläche 20 zugewandt. Die Unterseite der Grundfläche ist als Auflagefläche zur Auflage auf der Körperfläche 20 ausgebildet. Die Grundfläche umgibt die Sensoreinheit 10 vollumfänglich und ist in seiner Aussenkontur auf die Sensoreinheit 10 abgestimmt, wobei insbesondere Flügel vorgesehen sind, welche weitgehend senkrecht zu beiden Seiten der Längsachse 18 vorgesehen sind. Die Form der Sensormatte 12 bestimmt die Grösse der Auflagefläche und somit die Auflage auf der Körperoberfläche 20 zur Befestigung der Sensoranordnung 1. Auf der Auflagefläche der unteren Schicht 13 ist eine Haftlage angeordnet.

Die Oberseite der unteren Schicht 13 steht in Kontakt mit der oberen Schicht 14 der mehrschichtigen Sensormatte 12, insbesondere sind untere Schicht 13 und obere Schicht 14 an der Kontaktfläche miteinander lösbar verbunden, beispielsweise durch eine Klebeschicht. Die obere Schicht 14 weist ebenfalls eine Öffnung 17 auf, durch welche der Kontaktkopf 16 zumindest teilweise geführt ist. Demnach überdeckt die obere Schicht 14 der Sensormatte 12 als eine Abdeckung die Sensoreinheit 10. Die Sensormatte 12 ist flexibel ausgebildet, um sich der Kontur der Körperoberfläche 20 anpassen zu können.

Die Sensormatte 12 ist als einmal verwendbare Einheit vorgesehen und wird nach einem einmaligen Gebrauch entsorgt. Die mehrschichtige Sensormatte 12 weist mindestens eine Schaumschicht auf.

In Figur 2a ist eine schematische Aufsicht auf die Sensoreinheit 10 dargestellt, welche als flexible oder starr-flexible Leiterplatte ausgebildet ist. Die Sensoreinheit 10 ist in Form eines sich entlang der Längsachse 18 erstreckenden flachen Körpers ausgebildet, wobei erste und zweite Lichtdetektoreinrichtungen 2, bzw. Sensorflächen 2, angeordnet sind. Die Lichtdetektoreinrichtungen 2 sind von einer Lichteinkopplungsstelle an einer Kontaktfläche 22, bzw. Lichtaustrittsfläche oder Lichteinkopplungsstelle, in unterschiedlichen Trennabständen positioniert. Der Trennabstand richtet sich nach den Begebenheiten der Anwendung und bezieht sich in der dargestellten Ausführungsform auf Lichtdetektoreinrichtungen 2 zur Messung in Körperoberflächen 20 nahen und tieferen Körpergeweben. Entlang der Längsachse 18 erstrecken sich Leiterbahnen 5, welche sowohl zur Signalleitung als auch zur elektrischen Kontaktierung eingerichtet sind und ein erstes Kontaktmittel 30 mit einer Kontaktgeometrie 32 und. Das erste Kontaktmittel 30 ist weitgehend ringförmig integral an der als flexible oder starr-flexible Leiterplatte ausgebildeten Sensoreinheit 10 ausgebildet. Dabei sind an einem oder mehreren Bereichen an einem Aussenumfang des weitgehend ringförmigen ersten Kontaktmittels 30 Positionierelemente 31 vorgesehen, beispielsweise in Form von Einstülpungen am Umfang. Die Formgebung des ringförmigen ersten Kontaktmittels 30 ist komplementär einer Formgebung eines Positionierrings 33 ausgebildet, der in Figur 2b dargestellt ist. Der Positionierring 33 weist an einem Innenumfang die entsprechend ausgebildeten Positionierelemente 35 auf, beispielsweise Ausstülpungen, welche erlauben, den Positionierring 33 in einer vorgegebenen Orientierung an der Sensoreinheit 10 zu positionieren und zu fixieren. Der Positionierring 33 erlaubt, dass der mit der Sensoreinheit 10 zu kontaktierende Kontaktkopf 16 durch die Formgebung des Positionierrings 33 in einer vorgegebenen Orientierung positionierbar ist. Auf einer Ringfläche des ersten Kontaktmittels 30 sind magnetische Kontaktelemente 34 und elektrische Kontaktelemente 36 in einer Anordnung gemäss der Kontaktgeometrie 32 angeordnet. Die elektrischen Kontaktelemente 36 können elektrisch leitend zum Aktivieren der Lichtdetektoreinrichtungen 2 via der Leiterbahnen 5 kontaktiert werden. Die magnetischen Kontaktelemente 34 bilden mit einem entsprechenden Gegenstück, vorgesehen am Kontaktkopf 16, eine lösbare Verbindung der Sensoreinheit 10 mit dem Kontaktkopf 16, wie dies noch beschrieben wird. Durch die magnetische Kopplung wird die elektrische Kontaktierung der Sensoreinheit 10 bereitgestellt.

An dem langgestreckten Körper der Sensoreinheit 10 sind im Bereich der optischen Komponenten, bzw. der Sensorflächen 2, als auch an der Lichteinkopplungsstelle 22 Laschen 38, bzw. Nasen, ausgebildet, welche sich senkrecht und/oder entlang der Längsachse 18 erstrecken. Die angeformten Laschen 38 bilden eine Auflagefläche auf einer Fläche der mehrschichtigen Sensormatte 12. In der dargestellten Perspektive nicht erkennbar, weist die als flexible oder starr-flexible Leiterplatte ausgebildete Sensoreinheit 10 Verstärkungselemente 39 auf, welche beispielsweise als Materialverdickungen an den Positionen der optischen Komponenten ausgebildet sein können.

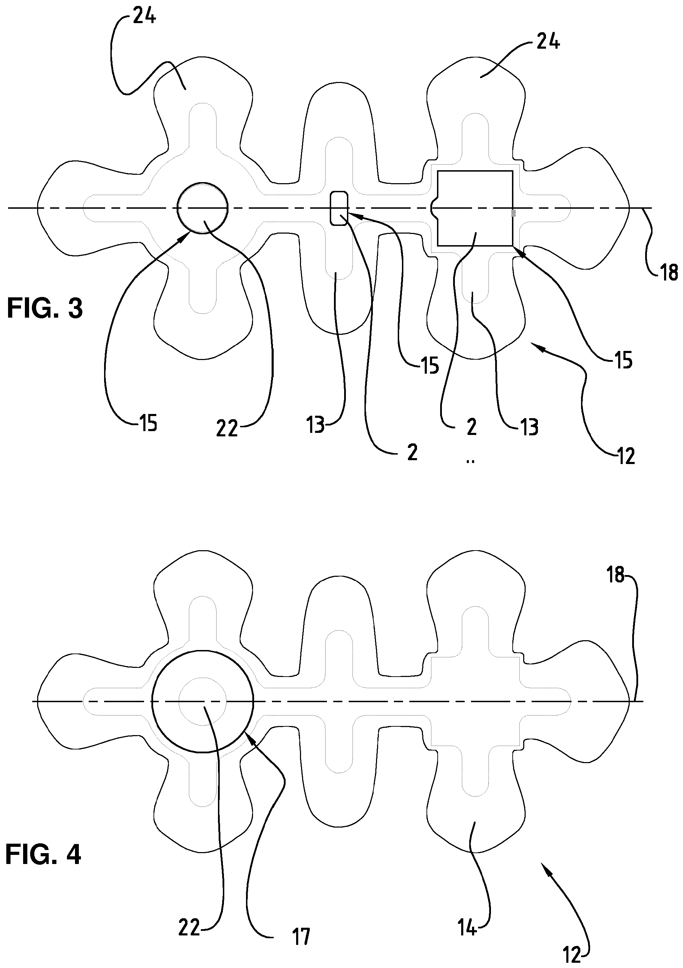

In Figur 3 ist eine Aufsicht auf die Sensormatte 12 von unten aus betrachtet dargestellt, so dass die Grundfläche der unteren Schicht 13 sichtbar ist. Die Sensormatte 12 ist als länglicher flacher Körper ausgebildet, welcher sich insbesondere entlang der Längsachse 18 erstreckt. Die Grundfläche, welche die Auflagefläche zur Auflage auf der Körperoberfläche 20 bildet, weist mehrere Aussparungen 15 in den Bereichen auf, an welchen optische Komponenten der Sensoreinheit 10 vorgesehen sind, beispielsweise die Lichteinkopplungsstelle 22 und die Lichtdetektoreinrichtungen 2. Die Aussenkontur der Sensormatte 12 kann unterschiedliche Formen haben, insbesondere können in Bereichen der optischen Komponenten Flügel vorgesehen sein, welche allgemein mit 24 bezeichnet werden und welche sich zumindest teilweise senkrecht zur Längsachse 18 erstrecken.

In Figur 4 ist eine Aufsicht auf die obere Schicht 14 der Sensormatte 12 gezeigt, welche in Form der Aussenkontur weitgehend derjenigen der unteren Schicht 13 entspricht. Allerdings ist an der oberen Schicht 14 lediglich eine Öffnung 17 im Bereich der Kontaktfläche 22 bzw. der Lichteinkopplungsstelle 22 ausgebildet, welche einen Durchgang für einzustrahlendes Licht in die darunter befindliche Körperoberfläche 20 bereitstellt.

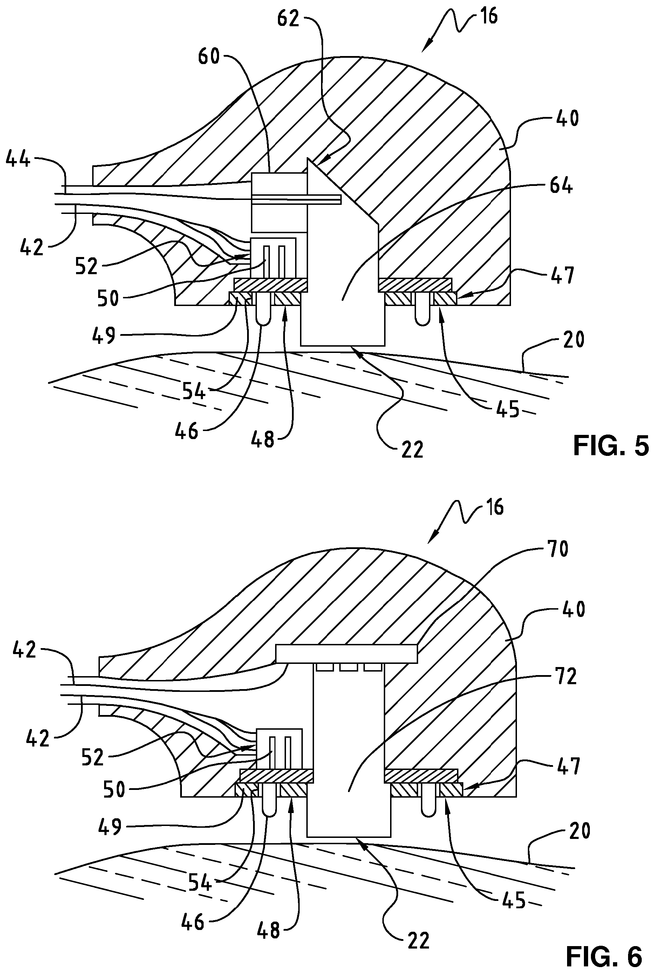

In Figur 5 ist eine Detailansicht als Schnittansicht eines Kontaktkopfes 16 gemäss einer ersten Ausführungsform dargestellt, welcher mit der Sensoreinheit 10 in operative Verbindung gebracht werden kann. Der Kontaktkopf 16 umfasst ein Gehäuse 40, in welches in einer nicht näher dargestellten Aufnahme elektrische Leitungen 42 und eine Faseroptik 44 aufnehmbar und fixierbar sind. Die elektrischen Leitungen 42 stehen in elektrisch leitender Verbindung mit Federkontaktstiften 46, welche an zweiten Kontaktmitteln 47 vorgesehen sind. In der dargestellten Ausführungsform ragen die Federkontaktstifte 46 nach unten aus einem ersten Kontaktring 48 der zweiten Kontaktmittel 47 vor und sind auf einer gegenüberliegenden Seite des ersten Kontaktrings 48 mittels Steckerelementen 50 elektrisch leitend mit den elektrischen Leitungen 42 verbunden, beispielsweise durch einen daran angeordneten Stecker 52. An dem ersten Kontaktring 48 kann ein zweiter Kontaktring 49 angeordnet sein, welcher Durchgangsbohrungen 54 aufweist, durch welche sich die Federkontaktstifte 46 erstrecken. Ferner sind an einer Unterseite des zweiten Kontaktrings 49 Magnetelemente bzw. magnetische Kontaktelemente 45 angeordnet, welche in komplementärer Anordnung zur Kontaktgeometrie 32 des ersten Kontaktmittels 30 (nicht dargestellt) positioniert sind.

Ferner ist in Figur 5 dargestellt, dass die Faseroptik 44 in einem in dem Gehäuse 40 aufnehmbaren Halteelement 60 aufgenommen und fixiert ist, wobei aus der Faseroptik 44 in einer Richtung austretendes Licht an einer bereitgestellten Reflexionsfläche 62 in eine zweite Richtung umgelenkt bzw. reflektiert wird, welche weitgehend senkrecht zur ersten Richtung orientiert ist und weitgehend senkrecht zur Körperoberfläche 20 durch die Kontaktfläche 22 bzw. Lichteinkopplungsstelle 22 austritt. Die Reflexionsfläche 62 wird gebildet durch ein schräges geschlossenes Ende eines als Zylinder ausgebildeten Umlenkelements 64, welches in dem Gehäuse 40 des Kontaktkopfes 16 aufgenommen und gehalten ist.

In Figur 6 ist eine alternative Ausführungsform des Kontaktkopfes 16 dargestellt, wobei insbesondere eine interne Lichtquelle 70 in Form von Leuchtdioden bzw. Laserdioden in dem Gehäuse 40 vorgesehen ist. Der Kontakt des Kontaktkopfes 16 zu der Kontaktgeometrie 32 des ersten Kontaktmittels 30 der Sensoreinheit 10 entspricht demjenigen der in Figur 5 dargestellten Ausführungsform. In Figur 6 ist schematisch die interne Lichtquelle 70 dargestellt, welche mittels elektrischer Leitungen 42 elektrisch kontaktiert werden kann. Das von der internen Lichtquelle 70 ausgehende Licht, vorzugsweise Lichtwellen mit unterschiedlichen Wellenlängen, wird entlang eines Lichtleiterelements 72 in Richtung Körperoberfläche 20 geführt. Das Lichtleiterelement 72 kann als ein Zylinder aus einem lichtleitenden Material ausgebildet sein, beispielsweise aus Polycarbonat und ist in dem Kontaktkopf 16 aufnehmbar.

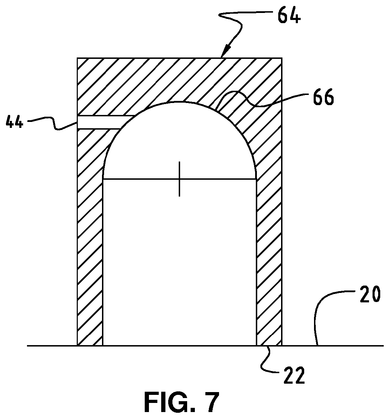

In Figur 7 ist eine Ausführungsform eines Umlenkelements 64 dargestellt. Das Umlenkelement 64 ist als separates Element ausgebildet, welches in den Kontaktkopf 16 aufnehmbar und darin fixierbar ist. In der dargestellten Ausführungsform ist das Umlenkelement 62 als Hohlzylinder mit einem halbkugelförmig geschlossenen Ende 66 ausgebildet. In dieses halbkugelförmige Ende 66 mündet eine Aufnahme für die Faseroptik 44, so dass aus der Faseroptik 44 austretendes Licht an der Halbkugelform reflektiert wird und im Wesentlichen senkrecht zur Faseroptik 44 umgelenkt wird, um aus der Lichteinkopplungsstelle 22 bzw. der Kontaktfläche 22 in die damit in Kontakt stehende Körperoberfläche 20 zu emittieren.

## Patentansprüche

1. Sensoranordnung (1) zur nicht-invasiven Messung von Parametern eines Körpergewebes mit einer Sensoreinheit (10) und einer Sensormatte (12) zum lösbaren Aufbringen der Sensoranordnung (1) auf einer Körperoberfläche (20), **dadurch gekennzeichnet, dass**
▪ die Sensoranordnung (1) einen Kontaktkopf (16) umfasst, ausgebildet zum elektrischen Kontaktieren der Sensoreinheit (10) und zum Zuführen von Licht von einer Lichtquelle zu der Sensoreinheit (10), wobei der Kontaktkopf (16) an der Sensoreinheit (10) aufnehmbar und mit dieser verbindbar ist,
▪ die Sensoreinheit (10) als eine flexible Leiterplatte ausgebildet ist;
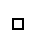 mit optischen Komponenten, umfassend mindestens eine Lichtdetektoreinrichtung (2), um Licht zu detektieren, welches von dem an der Sensoreinheit (10) aufgenommenen und verbundenen Kontaktkopf (16) in das Körpergewebe emittiert und dieses passiert hat, und
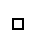 mit einem ersten Kontaktmittel (30) und Leiterbahnen (5) zum operativen Verbinden der Sensoreinheit (10) mit dem Kontaktkopf (16),
▪ und wobei die Sensormatte (12) einen mehrschichtigen Aufbau hat,
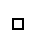 mit einer unteren Schicht (13), welche eine untere Auflagefläche zur Auflage auf der Körperoberfläche (20) und eine obere Auflagefläche zur Auflage der Sensoreinheit (10) aufweist, wobei Aussparungen (15, 17) vorgesehen sind, welche auf die optischen Komponenten der Sensoreinheit (10) abgestimmt sind, und einen optischen Durchgang bilden, und
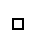 mit einer oberen Schicht (14), welche mit der oberen Auflagefläche der unteren Schicht (13) voneinander lösbar verbunden ist;
▪ und wobei die Sensoreinheit (10) als entlang einer Längsachse (18) gestreckter Körper ausgebildet ist,
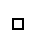 und wobei an dem langgestreckten Körper der Sensoreinheit (10) im Bereich der optischen Komponenten, bzw. der Lichtdetektoreinrichtung (2) und der Lichteinkopplungsstelle (22), Laschen (38) ausgebildet sind, welche sich senkrecht zur und/oder entlang der Längsachse (18) erstrecken.

2. Sensoranordnung (1) nach Anspruch 1, **dadurch gekennzeichnet, dass** die Sensoreinheit (10) zumindest abschnittsweise Verstärkungselemente (39) aufweist.

3. Sensoranordnung (1) nach einem der vorhergehenden Ansprüche, **dadurch gekennzeichnet, dass** das erste Kontaktmittel (30) magnetische Kontaktelemente (34) und elektrische Kontaktelemente (36) umfasst.

4. Sensoranordnung (1) nach Anspruch 3, **dadurch gekennzeichnet, dass** die magnetischen Kontaktelemente (34) und die elektrischen Kontaktelemente (36) des ersten Kontaktmittels (30) der Sensoreinheit (10) gemäss einer Kontaktgeometrie (32) angeordnet sind, welche zum elektrischen Kontaktieren und zum Verbinden mit dem Kontaktkopf (16) ausgebildet ist.

5. Sensoranordnung (1) nach einem der vorhergehenden Ansprüche, **dadurch gekennzeichnet, dass** der Kontaktkopf (16) umfasst:
▪ ein Gehäuse (40),
▪ mindestens zweite Kontaktmittel (47), welche eine Kontaktgeometrie (32) entsprechend dem ersten Kontaktmittel (30) der Sensoreinheit (10) aufweisen,
▪ elektrische Leitungen (42), welche in dem Gehäuse (40) fixierbar und welche mit elektrischen Kontaktelementen (46) der zweiten Kontaktmittel (47) leitend verbindbar sind,
▪ Mittel (44, 64; 70, 72) zum Bereitstellen und Führen von Licht in einer Richtung, welche senkrecht zu der Körperoberfläche (20) gerichtet ist, wenn die Sensoranordnung (1) auf der Körperoberfläche (20) angebracht ist, und
▪ eine Lichtaustrittsfläche (22), über welche das in der Richtung geführte Licht in das Körpergewebe emittierbar ist.

6. Sensoranordnung (1) nach Anspruch 5, **dadurch gekennzeichnet, dass** die zweiten Kontaktmittel (47) in dem Gehäuse (40) des Kontaktkopfes (16) als separate ringförmige Komponenten (48, 49) aufnehmbar sind, umfassend:
▪ einen ersten Kontaktring (48), an welchem auf einer ersten Seite die Federkontaktstifte (46) in einer vorgegebenen Anordnung und auf einer zweiten Seite Steckerelemente (52) angeordnet sind, welche mit einem an den elektrischen Leitungen (42) vorgesehenen Gegenstück einen Steckerkontakt (50) bilden, und
▪ einen zweiten Kontaktring (49), welcher Durchgangsbohrungen aufweist, durch welche sich die Federkontaktstifte (46) erstrecken und an welchem auf einer ersten Seite magnetische Kontaktelemente (45) angeordnet sind, welche mit denjenigen des ersten Kontaktmittels (30) in Kontakt bringbar sind.

7. Sensoranordnung (1) nach Anspruch 5 oder 6, **dadurch gekennzeichnet, dass** die Mittel (44, 64; 70, 72) eine Faseroptik (44) umfassen, um Licht einer externen Lichtquelle in einer ersten Richtung zu führen und ein Umlenkelement (64), welches das zugeführte Licht in die Richtung senkrecht zu der Körperoberfläche (20) umlenkt.

8. Sensoranordnung (1) nach Anspruch 7, **dadurch gekennzeichnet, dass** die Faseroptik (44) mindestens eine optische Faser umfasst, welche in dem Gehäuse (40) oder einem in dem Gehäuse (40) aufnehmbaren Element (60) fixiert ist, so dass Licht aus der Faseroptik (44) in das Umlenkelement (64) austritt.

9. Sensoranordnung (1) nach Anspruch 7 oder 8, **dadurch gekennzeichnet, dass** von dem in dem Gehäuse (40) aufnehmbaren Umlenkelement (64) eine Reflexionsfläche (62) bereitgestellt ist.

10. Sensoranordnung (1) nach Anspruch 9, **dadurch gekennzeichnet, dass** das Umlenkelement (64) als Zylinder mit einer geschlossenen abgeschrägten Endfläche als Reflexionsfläche (62) ausgebildet ist.

11. Sensoranordnung (1) nach Anspruch 10, **dadurch gekennzeichnet, dass** das Umlenkelement (64) als zumindest teilweise galvanisiertes Kunststoffelement gefertigt ist.

12. Sensoranordnung (1) nach einem der Ansprüche 7 bis 9,
**dadurch gekennzeichnet, dass** das Umlenkelement (64) als Hohlzylinder mit einer geschlossenen halbkugelförmigen Endfläche (66) als Reflexionsfläche (62) ausgebildet ist.

13. Sensoranordnung (1) nach Anspruch 12, **dadurch gekennzeichnet, dass** das Umlenkelement (64) aus einem reflektierenden Material gefertigt ist.

14. Sensoranordnung (1) nach Anspruch 5, **dadurch gekennzeichnet, dass** die Mittel (44, 64; 70, 72) als interne Lichtquelle (70) und als ein Lichtleiterelement (72) ausgebildet sind, welche in dem Gehäuse (40) des Kontaktkopfes (16) aufnehmbar sind.

15. Sensoranordnung (1) nach einem der Ansprüche 5 bis 14,
**dadurch gekennzeichnet, dass** in dem Kontaktkopf (16) eine Energiequelle vorgesehen ist zur elektrischen Kontaktierung via der elektrischen Leitungen (42).

16. Sensoranordnung (1) nach Anspruch 15, **dadurch gekennzeichnet, dass** eine drahtlose Datenübertragung der Sensoranordnung (1) mit einer externen Steuer- und Verarbeitungseinheit vorgesehen ist.

## Claims

1. Sensor configuration (1) for non-invasive measurement of parameters of a body tissue with a sensor unit (10) and a sensor mat (12) for detachable placement of the sensor configuration (1) on a body surface (20), **characterized in that**
▪ the sensor configuration (1) comprises a contact head (16), designed for electrical contacting of the sensor unit (10) and for supply of light from a light source to the sensor unit (10), whereby the contact head (16) is able to be received on the sensor unit (10) and is connectible thereto,
▪ the sensor unit (10) is designed as a flexible printed circuit board;
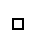 with optical components, comprising at least one light detector device (2), in order to detect light that has been emitted from the contact head (16), received on and connected to the sensor unit (10), into the body tissue and has passed through the latter, and
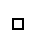 with a first contact means (30) and conducting tracks (5) for operative connection of the sensor unit (10) to the contact head (16),
▪ and whereby the sensor mat (12) has a multi-layered structure,
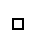 with a lower layer (13), having a lower contact surface for contact on the body surface (20) and an upper contact surface for support of the sensor unit (10), whereby openings (15, 17) are provided which are aligned with respect to the optical components of the sensor unit (10) and form an optical passage, and
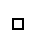 with an upper layer (14), which is connected to the upper contact surface of the lower layer (13) in a way detachable from one another;
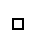 and whereby the sensor unit (10) is designed as a body stretched along a longitudinal axis (18),
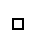 and whereby tabs (38) are formed on the longitudinally stretched body of the sensor unit (10) in the region of the optical components or the light detector device (2) and the light coupling point (22), which tabs extend perpendicular to and/or along the longitudinal axis (18).

2. Sensor configuration (1) according to claim 1, **characterized in that** the sensor unit (10) has, at least in sections, reinforcement elements (39).

3. Sensor configuration (1) according to one of the preceding claims, **characterized in that** the first contact means (30) comprises magnetic contact elements (34) and electrical contact elements (36).

4. Sensor configuration (1) according to claim 3, **characterized in that** the magnetic contact elements (34) and the electrical contact elements (36) of the first contact means (30) of the sensor unit (10) are arranged according to a contact geometry (32), which is designed for electrical contacting and for connecting to the contact head (16).

5. Sensor configuration (1) according to one of the preceding claims, **characterized in that** the contact head (16) comprises:
▪ a housing (40),
▪ at least two contact means (47), which have a contact geometry (32) corresponding to the first contact means (30) of the sensor unit (10),
▪ electrical lines (42), which are fixable in the housing (40) and which are connectible to electrical contact elements (46) of the second contact means (47) in a conductive way,
▪ means (44, 64; 70, 72) for providing and guiding of light in one direction, which is directed perpendicular to the body surface (20) when the sensor configuration (1) is placed on the body surface, and
▪ a light-emitting surface (22) via which the light guided in the direction is able to be emitted into the body tissue.

6. Sensor configuration (1) according to claim 5, **characterized in that** the second contact means (47) are able to be accommodated in the housing (40) of the contact head (16) as separate annular components (48, 49), comprising:
▪ a first contact ring (48), on which are disposed, on a first side, the spring contact pins (46) in a predetermined configuration, and, on a second side, connector elements (52), which form a plug contact (50) with a mating part provided on the electrical lines (42), and
▪ a second contact ring (49), which has through-holes, through which the spring contact pins (46) extend and on which, on a first side, magnetic contact elements (45) are disposed, which are able to be brought into contact with those of the first contact means (30).

7. Sensor configuration (1) according to claim 5 or 6, **characterized in that** the means (44, 64; 70, 72) comprise a fibre-optical element (44), in order to guide light from an external light source in a first direction and a deflector element (64), which deflects the supplied light in the direction perpendicular to the body surface (20).

8. Sensor configuration (1) according to claim 7, **characterized in that** the fibre-optical element (44) comprises at least one optical fibre which is fixed in the housing (40) or to an element (60) able to be accommodated in the housing (40), so that light exits out of the fibre-optical element (44) into the deflector element (64).

9. Sensor configuration (1) according to claim 7 or 8, **characterized in that** provided is a reflection surface (62) of the deflector element (64) able to be accommodated in the housing (40).

10. Sensor configuration (1) according to claim 9, **characterized in that** the deflector element (64) is designed as a cylinder with a closed beveled end face as reflection surface (62).

11. Sensor configuration (1) according to claim 10, **characterized in that** the deflector element (64) is manufactured at least in part as a galvanized plastic element.

12. Sensor configuration (1) according to one of the claims 7 to 9, **characterized in that** the deflector element (64) is designed as hollow cylinder with a closed semispherical end face (66) as reflection surface (62).

13. Sensor configuration (1) according to claim 12, **characterized in that** the deflector element (64) is made of a reflecting material.

14. Sensor configuration (1) according to claim 5, **characterized in that** the means (44, 64; 70, 72) are designed as internal light source (70) and as a light guide element (72), which are able to be accommodated in the housing (40) of the contact head (16).

15. Sensor configuration (1) according to one of the claims 5 to 14, **characterized in that** provided in the contact head (16) is an energy source for electrical contacting via the electrical lines (42).

16. Sensor configuration (1) according to claim 15, **characterized in that** wireless data transmission is provided for the sensor configuration (1) with an external control and processing unit.

## Revendications

1. Ensemble pour capteur (1) de mesure non invasive de paramètres d'un tissu corporel avec une unité de détection (10) et un tapis de capteur (12) pour le positionnement amovible de l'ensemble pour capteur (1) sur une surface corporelle (20), **caractérisée en ce que**
▪ l'ensemble pour capteur (1) comprend une tête de contact (16), conçue pour le contact électrique de l'unité de détection (10) et pour l'alimentation en lumière de l'unité de détection (10) à partir d'une source de lumière, la tête de contact (16) pouvant être disposée sur l'unité de détection (10) et y être connectée,
▪ l'unité de détection (10) est agencée en tant que carte de circuit imprimé flexible ;
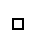 avec des composants optiques, comprenant au moins un dispositif détecteur de lumière (2), afin de détecter la lumière qui a été émise par la tête de contact (16), disposée sur et connectée à l'unité de détection (10), dans le tissu corporel et qui a traversé ce dernier, et
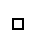 avec un premier moyen de contact (30) et des pistes conductrices (5) pour la connexion opérationnelle de l'unité de détection (10) à la tête de contact (16),
▪ et le tapis de capteur (12) possédant une structure multicouche,
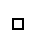 avec une couche inférieure (13), ayant une surface de contact inférieure pour le contact avec la surface du corps (20) et une surface de contact supérieure pour le support de l'unité de détection (10), dans laquelle des ouvertures (15, 17) sont prévues et qui sont alignées par rapport aux composants optiques de l'unité de détection (10) et forment un passage optique, et
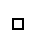 avec une couche supérieure (14), qui est reliée à la surface de contact supérieure de la couche inférieure (13) de façon amovible l'une par rapport à l'autre ;
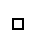 et l'unité de détection (10) étant conçue comme un corps étiré le long d'un axe longitudinal (18),
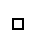 et des languettes (38) étant formées sur le corps étiré longitudinalement de l'unité de détection (10) dans la zone des composants optiques ou du dispositif de détection de lumière (2) et du point de couplage de lumière (22), ces languettes s'étendant perpendiculairement à l'axe longitudinal (18) et/ou le long de cet axe.

2. Ensemble pour capteur (1) selon la revendication 1, **caractérisé par le fait que** l'unité de détection (10) comporte, au moins en partie, des éléments de renforcement (39).

3. Ensemble pour capteur (1) selon l'une des revendications précédentes, **caractérisé en ce que** le premier moyen de contact (30) comprend des éléments de contact magnétiques (34) et des éléments de contact électriques (36).

4. Ensemble pour capteur (1) selon la revendication 3, **caractérisé en ce que** les éléments de contact magnétiques (34) et les éléments de contact électriques (36) des premiers moyens de contact (30) de l'unité de détection (10) sont disposés selon une géométrie de contact (32), qui est conçue pour réaliser le contact électrique et réaliser la connexion avec la tête de contact (16).

5. Ensemble pour capteur (1) selon l'une des revendications précédentes, **caractérisé en ce que** le la tête de contact (16) comprend:
▪ un boîtier (40),
▪ au moins deux moyens de contact (47), qui ont une géométrie de contact (32) correspondant au premier moyen de contact (30) de l'unité de détection (10),
▪ des lignes électriques (42), qui peuvent être fixées dans le boîtier (40) et qui peuvent être connectées aux éléments de contact électrique (46) des seconds moyens de contact (47) de manière conductrice,
▪ des moyens (44, 64; 70, 72) pour fournir et guider la lumière dans une direction, qui est dirigée perpendiculairement à la surface du corps (20) lorsque l'ensemble pour capteur (1) est placé sur la surface du corps, et
▪ une surface d'émission de lumière (22) par laquelle la lumière guidée dans la direction peut être émise dans le tissu corporel.

6. Ensemble pour capteur (1) selon la revendication 5, **caractérisé en ce que** les seconds moyens de contact (47) peuvent être logés dans le boîtier (40) de la tête de contact (16) en tant que composants annulaires séparés (48, 49), comprenant :
▪ un premier anneau de contact (48), sur lequel sont disposés, sur un premier côté, les éléments de contact électriques (46) sont constitués par des broches de contact à ressort dans une configuration prédéterminée, et, sur un second côté, des éléments de connexion (52), qui forment un contact d'enfichage (50) avec une partie correspondante prévue sur les lignes électriques (42), et
▪ un deuxième anneau de contact (49), qui présente des trous traversants, à travers lesquels s'étendent les broches de contact à ressort (46) et sur lequel sont disposés, sur un premier côté, des éléments de contact magnétiques (45) qui peuvent être mis en contact avec ceux du premier moyen de contact (30).

7. Ensemble pour capteur (1) selon la revendication 5 ou 6, **caractérisé par le fait que** les moyens (44, 64 ; 70, 72) comprennent une fibre optique (44), afin de guider la lumière provenant d'une source lumineuse externe dans une première direction, et un élément déflecteur (64), qui dévie la lumière fournie selon une direction perpendiculaire à la surface du corps (20).

8. Ensemble pour capteur (1) selon la revendication 7, **caractérisé par le fait que** l'élément à fibre optique (44) comprend au moins une fibre optique fixée dans le boîtier (40) ou à un élément (60) pouvant être logé dans le boîtier (40), de sorte telle que la lumière ressorte de l'élément à fibre optique (44) vers l'élément déflecteur (64).

9. Ensemble pour capteur (1) selon la revendication 7 ou 8, **caractérisé par le fait qu'**une surface de réflexion (62) de l'élément déflecteur (64) peut être logée dans le boîtier (40).

10. Ensemble pour capteur (1) selon la revendication 9, **caractérisée par le fait que** l'élément déflecteur (64) est conçu comme un cylindre dont la surface de réflexion (62) est une face terminale fermée et biseautée.

11. Ensemble pour capteur (1) selon la revendication 10, **caractérisé par le fait que** l'élément déflecteur (64) est fabriqué au moins en partie comme un élément en plastique galvanisé.

12. Ensemble pour capteur (1) selon l'une des revendications 7 à 9, **caractérisée par le fait que** l'élément déflecteur (64) est conçu comme un cylindre creux avec une surface d'extrémité semi-sphérique fermée (66) comme surface de réflexion (62).

13. Ensemble pour capteur (1) selon la revendication 12, **caractérisé par le fait que** l'élément déflecteur (64) est constitué d'un matériau réfléchissant.

14. Ensemble pour capteur (1) selon la revendication 5, **caractérisé par le fait que** les moyens (44, 64 ; 70, 72) sont conçus comme une source de lumière interne (70) et comme un élément de guidage de la lumière (72), qui peuvent être logés dans le boîtier (40) de la tête de contact (16).

15. Ensemble pour capteur (1) selon l'une des revendications 5 à 14, **caractérisé par le fait que** la tête de contact (16) contient une source d'énergie pour le contact électrique via les lignes électriques (42).

16. Ensemble pour capteur (1) selon la revendication 15, **caractérisé par le fait que** la transmission de données sans fil est assurée pour l'ensemble pour capteur (1) avec une unité de commande et de traitement externe.
